# EUROPEAN PATENT APPLICATION

(11) **EP 1 746 422 A1**
(43) Date of publication of application: **24.01.2007**
(21) Application number: 05015597.7
(22) Date of filing: 19.07.2005
(51) Int. Cl.: G01N 33/68, G01N 33/53, G01N 33/566

(54) **Method for the identification of proteins folding inhibitors**

(71) Applicant: UNIVERSITA'DEGLI STUDI DI MILANO, 20122 Milano (IT)
(72) Inventor: Broglia, Ricardo, 20133 Milano (IT); Tiana, Guido, 20129 Milano (IT)
(74) Representative: Long, Giorgio

(57) **Abstract**

The present invention relates to a method for the identification of peptide inhibitors of the folding and thus of the biological function(s) of proteins which do not create resistance. In particular, the invention relates to inhibitors of viral enzymes with a high mutation rate.

## Description

The present invention relates to a method for the identification of inhibitors of the folding and thus of the biological function(s) of proteins and, more in particular, of peptidic inhibitors of the folding of proteins which are highly selective and which do not create resistance.

### Background

The fact that proteins play a primary physiological role is well known in the art. Many efforts have been taken to employ proteins as therapeutic agents, as catalysts and also as suitable materials possessing specific properties.

Many diseases stem from mutations in proteins that cause them to loose functionality. In some cases, for instance, the catalytic activity exerted by proteins may be impaired thus resulting in an altered metabolic pathway (e.g., phenylketonuria). In some other cases, structural properties of the proteins themselves may be affected so as to lead to a loss of physical functionality (e.g., muscular dystrophy). Creutzfeld-Jakob disease and other transmissible encephalopathies may result from structural modifications of proteins changing their shape and forming polymers [1]. Similarly, diseases may also result from amyloidosis in which proteins gradually convert into long chains of polymerized beta-sheets and precipitate to form fibrils [2].

Many cancers are known to occur because of mutations of proteins. To this extent, approximately 50% of human cancers are known to be caused by mutations in the tumor supressor P53 factor that primarily lowers its stability [3].

Enzymes and receptors are the usual targets of drugs, either to restore function or to destroy infectious agents or cancers. The ultimate goal of protein science is to be able to predict both the structure and activity of proteins from their amino acid sequence (the so-called "folding problem") as well as to inhibit this activity [4,5].

With this achievement it will be possible to design and synthesize novel catalysts, materials and pharmacologically active agents, in particular drugs suitable to inhibit enzymatic activity.

The main properties these drugs may display are specificity (i.e., not be toxic) and efficiency. Conventionally, this may be achieved by either capping the active site of the enzyme (competitive inhibition) or through binding onto some other regions/parts of the protein, thus provoking structural changes that make the enzyme unsuitable for binding to the substrate (allosteric inhibition).

To achieve any of these goals, the binding between the ligand and the protein has to be optimized. This is a rather formidable, time consuming problem, due to the fact that it is necessary to calculate not only the energies between enzymes and substrate or other ligands but, also, their interaction energies with water and the change of entropy during the reaction. The net binding energies are the small differences between two larger numbers.

A further complication arises in the case the target is a viral protein displaying a high mutation rate, often associated with the well-known development of resistance.

Therefore, it is of crucial importance to devise novel strategies that allow a more efficient and economic design of protein inhibitors over the known active-site centered designs, as well as to generate strategies aimed at blocking the interaction between enzymes and their substrate which do not generate resistance.

A number of experimental [6-8,38,41] and theoretical studies [9,10] suggest that globular, single-domain proteins (i.e., proteins of length N comprised between 60 and 150 amino acids [28]) fold through a hierarchical mechanism, where small units composed of few consecutive amino acids build larger units which, in turn, build even larger ones, which eventually involve the whole protein.

Experimental studies of the folding and association of amino acid chain segments, prior to native-state formation, have identified partial native-like structures among the initial folding events [39]. These structural elements are commonly referred to as folding domains or foldons [9]. The operational definition of these units is: "the three dimensional structure of the first observable native-like structures that the protein folds into, starting from a denaturated state". Mutations within those structural domains can severely limit the formation of a properly folded protein [10].

Model calculations [11,12] have shown that the folding of small, monomeric, single domain proteins proceed, starting from an unfolded conformation, following a hierarchical-succession of events: 1) formation of few (2-4) local elementary structures (commonly referred to as LES) containing on the whole from about 20% to about 30% of the protein's amino acids (and thus between 5% and 15% of the protein's amino acids each), stabilized by few highly conserved, strongly-interacting ("hot") hydrophobic amino acids (<10% of the protein's amino acids) lying close along the polypeptide chain; 2) docking of the LES in the (postcritical) folding nucleus [13], that is formation of the minimum set of native contacts which brings the system over the major free energy barrier of the whole folding process; 3) relaxation of the remaining amino acids onto the native structure shortly after the formation of the folding nucleus. The "hot" sites which stabilize the LES are found to be very sensitive to (non-conservative) point mutations. Since most of the protein stabilization energy is concentrated in these sites, the possibility of mutating one or two of them has a high probability of destabilizing the folded conformation. It is natural to identify the folding domains of the previous paragraph with the LES of model calculations.

The same model indicates that it is possible to destabilize the native conformation of a protein making use of peptides (which we shall call p-LES) whose sequence is identical to that of the protein's LES [14].

There are two important advantages of these folding-inhibitors with respect to conventional ones. First, their molecular structure is suggested directly by the target protein. One has not to design or to optimize anything, just find the LES of the target protein. Because the design of the LES has been performed by evolution through a myriad of generations of the virus (or of the organism which expresses the protein), to recognize and strongly interact with each other so as to make the protein fold fast as well as to avoid aggregation with other proteins, the resulting inhibitors are expected to display little toxicity. Second, it is unlikely that it can be rendered non-operative through escape mutants. In fact, p-LES bind to the complementary LES of the target protein, following the same paradigm which stabilizes the folding nucleus, stabilization which is controlled by the "hot" amino acids of the protein[15,16].

Consequently, escape mutants must contain mutations on those "hot" amino acids which are essential for the stabilization and docking of LES. Such mutations lead, as a rule, to protein denaturation. In other words, structural mutations which do not prevent the protein from folding to its native, biologically active state, do not prevent neither the docking of the local elementary structures into the folding nucleus, nor the inhibitor action of the p-LES. Mutations which prevent the formation of the folding nucleus, either by destabilizing LES or their docking may, in principle, avoid the action of the p-LES, but will not be expressed because of the inability of the mutated protein to fold.

### Summary of the invention

To sum up, the invention relates to a simple, economic and (essentially) error-free method to individuate the LES of globular, single-domain proteins (typical length of these proteins being from 60 to 150). Consequently, it relates to the individualization of highly-specific, strongly efficient inhibitors of the folding of these proteins (p-LES peptides) which are unlikely to create resistance.

Because globular, multi-domain proteins are, as a rule, constructed as a combination of sequence units (domains, blocks [17-24], or modules [25,26]) of characteristic length of about 125 amino acids for eukariotes and about 150 amino acids for prokariotes [27,28]), sequence units which fold as single domain proteins do, the invention relates also to the method for identifying peptide inhibitors of the folding of proteins regardless of their size or modularity, as well as to each of the monomers of three-state multimers [29,30].

In what follows we explain the invention within the framework of sequence units of a globular protein or of a monomer belonging to a three-state multimer.

### Object of the invention

The present invention therefore refers to a method for the identification of peptidic inhibitors of the folding, and thus of the specific biological activity [4], of proteins without inducing escape mutants.

It is therefore a first object of the present invention a method for the identification of peptide inhibitors of the folding of a protein containing N amino acids, which method comprises:
a) designing M peptides of length L, each displaying a sequence identical to a segment of the target protein, so as to cover the entire protein, allowing for some amount of overlap between the different peptides. Typically L contains about 10 amino acids and, preferably, varies from about 4 to about 20. Consequently, M ranges from about 5 to about 50, typically being about 20.
b) preparing the M designed peptides either singularly or in groups;
c) preparing M solutions each containing the protein under consideration and one of the peptides in a suitable molar ratio and incubating each of the solutions at 37°C.
d) assessing the inhibitory efficiency of the peptide in the above solutions or the degree of unfolding or both, by means of standard techniques, so as to identify the peptide endowed with inhibitory activity towards the protein.

The method of the present invention is particularly advantageous as it enables to identify in a reliable and rather simple way the peptide, among the M peptides purposely designed and prepared, that possesses an inhibitory activity, or even the highest inhibitory activity, towards the protein under consideration.

### Detailed description of the invention

According to the present invention, unless otherwise provided, with the term "peptide(s)" is meant short peptidic chain(s) comprising, typically, of the order of ten amino acids. Preferably, the peptide comprises L amino acids with L varying from about 4 to about 20. The sequence of the "peptide(s)", unless otherwise defined, coincides with a segment of equal length of the protein.

Furthermore, by "peptidic-inhibitor" we intend a peptide which blocks the folding of the target protein and, hence, its specific biological function. This is to say that the inhibitor has a sequence essentially identical to that of a LES of the protein, and can thus be also called a p-LES [14].

By "LES" (Local Elementary Structure) we intend the first native structures formed very early in the folding process [11,12] (also called foldons or folding domains in the literature [9,10,38]). These structures are stabilized by strongly interacting, as a rule hydrophobic, highly conserved ("hot") amino acids. One can distinguish between well structured, so called closed LES and less structured, so called, open LES [15]. This last type of LES do not display, when isolated in the solvent, any (important) native contact. On the contrary, closed LES, when isolated do display native contacts which play an important role also in the (postcritical) folding nucleus [13].

By (post-critical) folding nucleus "FN" we intend the minimum set of native contacts needed to overcome the highest free energy barrier encountered by the protein in the whole folding process [13]. A large fraction of these contacts arise from the docking of the LES. This event is essentially controlled by the closed LES.

By "hot" amino acids we intend amino acids which play a central role in the folding of a protein. Non-conservative mutation of these amino acids lead, as a rule, to protein denaturation [16].

According to step (a) of the present method, together with any variant thereof, M peptides of length L may be thus designed displaying a sequence identical to a segment of the protein under consideration. The values of M and L are chosen so as to cover the entire amino acid sequence of the protein also allowing for some amount of overlap. Typically L is 10 amino acids (corresponding, for single-domain proteins of length N, to approximately one tenth of the total number of amino acids N, that is, L□N/10) and, preferably, varies from about 4 to about 20. Consequently, M ranges from about 5 to about 50, typically being about 20.

Just as a non limiting explanatory example, the present method may provide for the identification of peptides possessing inhibitory activity towards a protein, e.g. comprising 120 amino acids, by designing 19 peptides (i.e. M equal to 19) each having an amino acid chain length of 12 amino acids.

From the above, as the 19 peptides each having a length of 12 amino acids display a sequence identical to given segments of the protein and as the whole of the protein sequence has to be covered, it is clear to the skilled person that peptides with overlapping segments will occur (the overlap between each consecutive peptide being in this particular example about 50%).

Typically, step (a) of the present method may provide for systematically designing the given M peptides by starting from amino acid number one and following, so as to progressively cover the entire protein (hence allowing for about 50% to about 70% of overlapping between the different peptides; see following Example 4).

Alternatively, the said M peptides may be designed by starting from those regions corresponding to the protein segments close to the nitrogen and the carbon terminals as well as to the centre of the protein, allowing again for overlapping among the different peptides.

Step (b) comprises preparing the M peptides according to methods well known in the art. The said methods may include, as an example, any known synthetic approach for the synthesis of the peptides or, alternatively, the possibility of taking them directly from the protein, by cutting it at the appropriate sites, as per known methods.

Step (c) is carried out by dissolving any of the peptides being prepared in step (b) together with the target protein in any suitable solvent. The molar ratio between the peptide and the protein may be properly varied in the range from 1:1 to 10:1 (peptide/protein); preferably, the relative concentration peptide/protein is 3 to 1.

The thus obtained solutions are incubated at about 37°C for a few minutes, for instance up to 10 minutes.

Alternatively, steps b) and c) can be substituted by computer simulations where use is made of simplified models of proteins (e.g., all-atom Gⓞ model, C_{α} Gⓞ-model, etc. [33]) coupled with thermodynamical sampling or simulated dynamics (e.g., Monte Carlo algorithm, Newton Dynamics, etc.) to simulate the evolution of the protein in the presence of each type of peptide (starting from either unfolded or from the folded conformations of the protein) and thus determine the degree of unfolding of the protein itself in the presence of these peptides.

These simulations can provide information not only on the sequence of the inhibitors, being identical to a segment of the protein, but also on their native conformation in the solvent. Those displaying a high degree of structure and stability are to be preferred as compared to inhibitors displaying little structure and/or large fluctuations. This in keeping with the fact that highly structured inhibitors (corresponding to the so called "closed" local elementary structures (LES), i.e. segments of motives displaying native contacts important in the stability of the protein), are expected to be more specific and thus less toxic than other less structured, so called "open" LES [11,12,15]. In this respect it is worth noting that model calculations which only consider the C_{α} -atom of the residues, emphasize the differences existing between these two types of LES.

When systematic experimental information exists concerning the ϕ-values [4] associated with a large number of sites of the protein, so as to allow for a determination of the "warm" and "hot" sites of the protein (i.e. those sites which play a central role in the folding of the protein [16], the corresponding amino acids occupying them being highly conserved), one can eventually slightly adjust the length and the number of initial and final amino acids along the protein so as to guarantee that the peptide inhibitors include all of the hot amino acids and most, if not all, of the warm amino acids.

The iteration process can always be carried out by calculating the ϕ-values associated with the different amino acids of the target protein, making use of an appropriate software (see following Example 1). Once the new inhibitors have been designed, it is also possible to test their efficiency through the assays as per step (d) above.

According to step (d) of the present method of the invention, the inhibitory efficiency of the peptide or the degree of unfolding or both, in the above solutions, may be carried out according to conventional methods. As a non limiting example, spectrophotometric assays, sedimentation equilibrium tests, circular dichroism or nuclear magnetic resonance techniques may be all employed and performed to determine the above inhibitory effect.

Likewise, experiments of absorption are known in the art [4] to determine the inhibitory efficiency when the protein is an enzyme [4,31].

As formerly reported, once found a peptide which efficiently blocks the folding of the protein, it is not a priori necessary to proceed with the process of checking the inhibitory properties of the remaining peptides, and the search and identification can be terminated at that point.

In other words,-the method of the invention may be applied to identify the optimal peptide endowed with the highest inhibitory activity, among all of those M peptide being prepared and so tested.

Alternatively, as above reported, the different solutions of step (c) can be tested in any order and the entire method stopped once a peptide having the desired inhibitory activity is found, without the need of testing and hence preparing all of the solutions.

As such, according to an alternative embodiment of the present invention, step (a) may comprise designing a single peptide of length varying from 4 to 20; step (b) may comprise preparing the said peptide; step (c) may comprise preparing a solution of the protein with that same peptide; step (d) may comprise assessing the inhibitory activity of the peptide. If the inhibitory efficiency of the said peptide is found as satisfactory, the method can be terminated as it easily allowed to identify the proper peptide inhibitor. On the contrary, if not satisfactory or anyway the peptide is devoid of any significant inhibitory efficiency, steps from (a) to (d) may be repeated with another peptide up to the identification of the optimal one.

The few (1-4) peptides which inhibit the folding of the protein, and which we call p-LES, identify the amino acid segments of the protein which, in the folding process, give rise to the LES.

Because a protein has to be, in order to fulfill its biological activity, in the native (folded) conformation, p-LES are also expected to be efficient, specific, perdurably effective inhibitors of the protein functions.

The p-LES should be tested for solubility in the culture medium, and can be modified to reduce their hydrophobicity, if needed.

Modification may for instance occur through: 1) addition of a polar and/or charged amino acids; 2) shortening the chain, that is loosing one or two hydrophobic amino acids at either C or N terminals, or both; 3) conservative mutations, that is by replacing an hydrophobic amino acid by another somewhat less hydrophobic amino acid.

In addition, as p-LES are peptides, they may be also digested by the cells and/or create allergies.

In this case, p-LES can be used as leads of folding inhibitors corresponding to their mimetic molecules or eventually peptides of the same amino acid sequence synthetized making use of D-amino acids, as per known methods.

Various aspects and embodiments of the present invention will now be described in more detail by way of examples, with reference also to peptide mimetics and solubility of p-LES.

The said examples are not to be intended as limiting the present invention as it will be appreciated that modifications of details of the method may apply without departing from the scope of the invention.

### Figures:

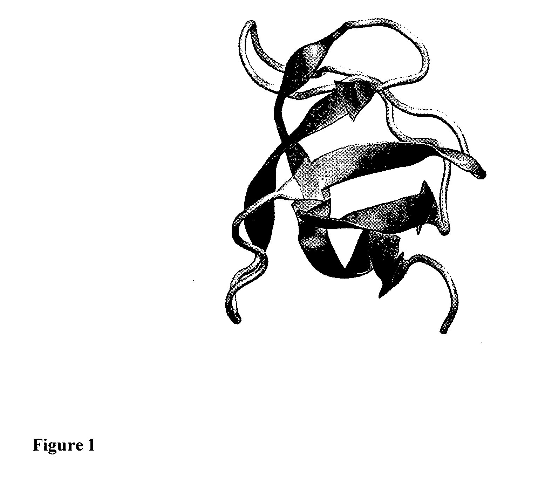

**Figure 1:** A cartoon of the crystallographic structure of src SH3. the (five) anti-parallel beta-sheets are depicted as arrowed (light gray) ribbons, while the alpha-helix is evidenced in dark gray.

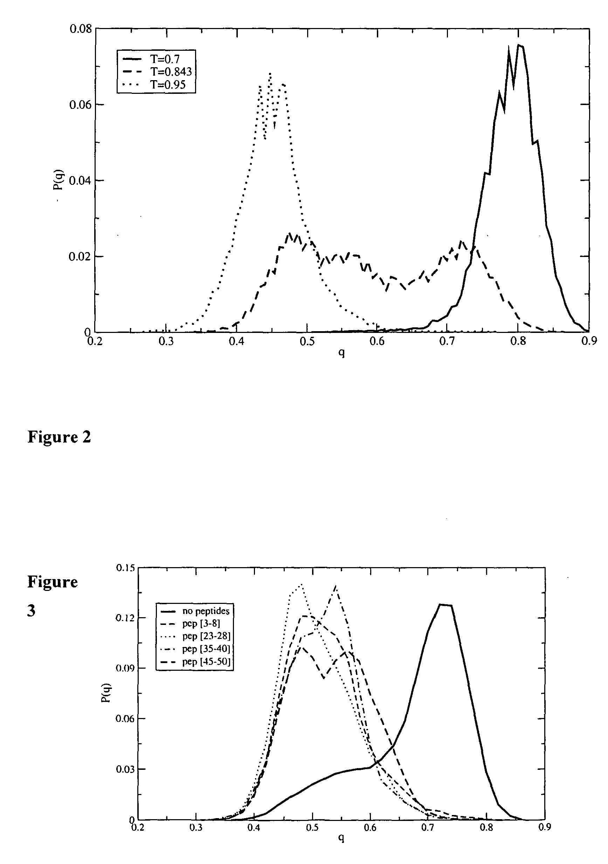

**Figure 2:** The equilibrium distribution probability of the order parameter q defined as the relative number of native contacts, calculated at three different temperatures for the SH3 protein alone. The protein is in the native conformation for values q>0.7 and in the denatured state for q<0.5. The temperature T=0.843 at which the peak of the native and denaturated states have the same area is the folding temperature.

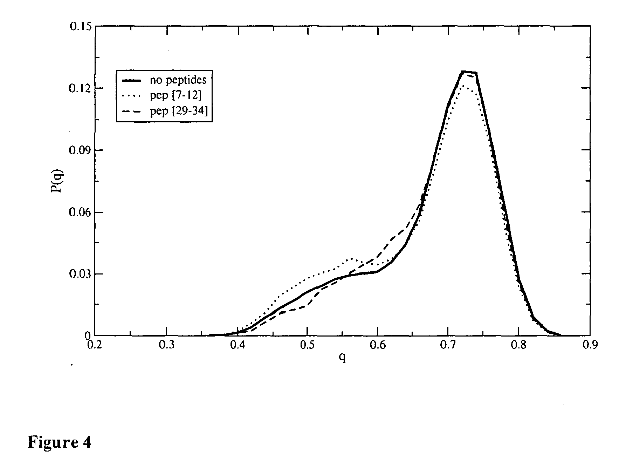

**Figure 3:** The equilibrium distribution probability at T=0.825 of the order parameter q for the system composed of the Src-SH3 protein domain and three peptides with the same sequence as one of the protein segments characterized by the first and last amino acids displayed in the inset.
**Figure 4:** The equilibrium distribution probability of the order parameter q calculated at T=0.825 for the system composed of the Src-SH3 protein domain and three peptides with the same sequence as one of the protein fragments characterized by the first and last amino acids displayed in the inset.

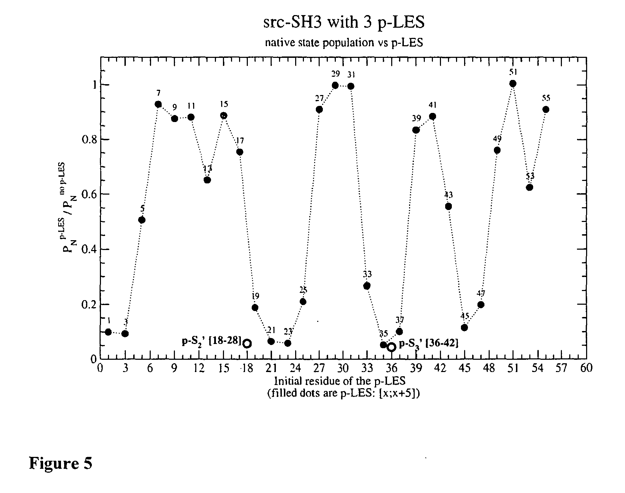

**Figure 5:** The population of the native state at T=0.825 for a system composed of the Src-SH3 protein domain and three peptides whose sequence is identical to that of the fragment of the protein of length 6 which starts at the site indicated on the abscissa, normalized with respect to the population of the native state of the protein by itself (p_{N}^{no-p-LES}). The peptides start with segment 1-6 and end with segment 55-60, the overlaps between the different peptides being 67%. The line is to guide the eye. The open dots provide information of the inhibitor properties of peptides p-S'₂ (=18-28) and p-S'₃ (=36-42) obtained by slight modification of two of the original peptides, namely peptides p-S₂ (=21-27) and p-S₃ (=35-40) (see text).

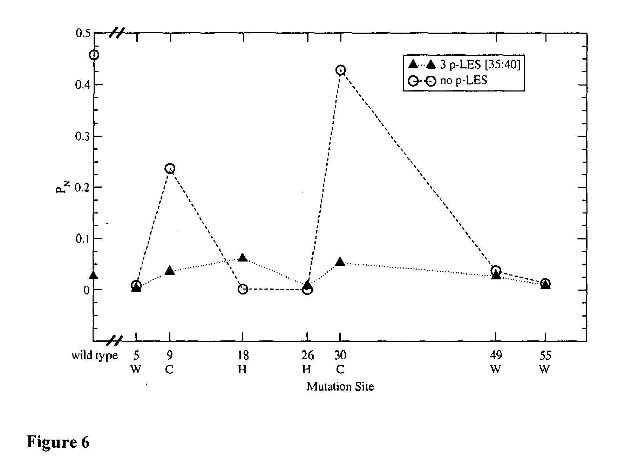

**Figure 6:** Population of the native state of Src SH3 protein domain (open dots) at T=0.825. The results displayed for the value 0 of the abscissa corresponds to the wild-type sequence. The other results correspond to the protein carrying a mutation on sites 9, 30 (cold, C), 5, 49 and 55 (warm, W), and 18, 26 (hot, H). The relative population of the native state of Src SH3 protein domain at T=0.825 in presence of three p-S₃ (35-40) is shown in terms of solid triangles. The lines are to guide the eye. The values at different mutation sites correspond to the wild-type sequence of the protein carrying the mutation indicated in the abscissa.

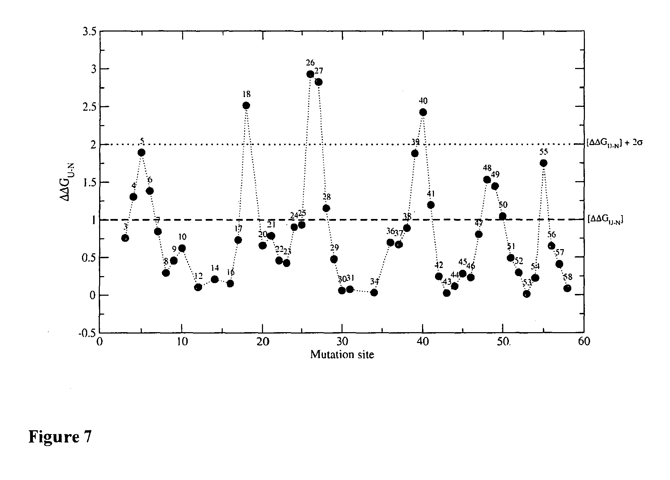

Figure 7: The change 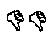 G in the free energy upon mutation of the Src-SH3 calculated making use of a modified Gⓞ model. The value of [ 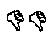 G] and [ 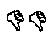 G]+2◆ are also displayed. The sites width 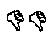 G > [ 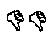 G]+2◆ (#18,26,27 and 40) are called hot amino acids, while those with [ 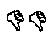 G] < 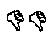 G < [ 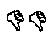 G]+2◆ (#4,5,6,28,39,41,48,49,50 and 55) qualify as warm amino acids. Systematic calculations and experimental information indicate that "hot" and "warm" sites of single-domain, monoglobular proteins, are present in the above mentioned ratios (cf. [40] and refs. therein).

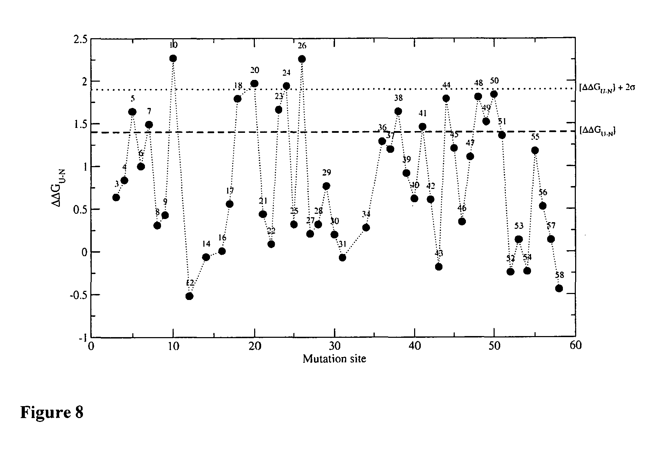

**Figure 8:** Same as Fig. 7 but for the experimentally determined □□G_{U-N} -values through protein engineering [32]. In this case, the sites 10, 20, 24 and 26 are hot sites, while sites 5, 7, 18, 23, 38, 41, 44, 48, 49 and 50 qualify as warm sites.

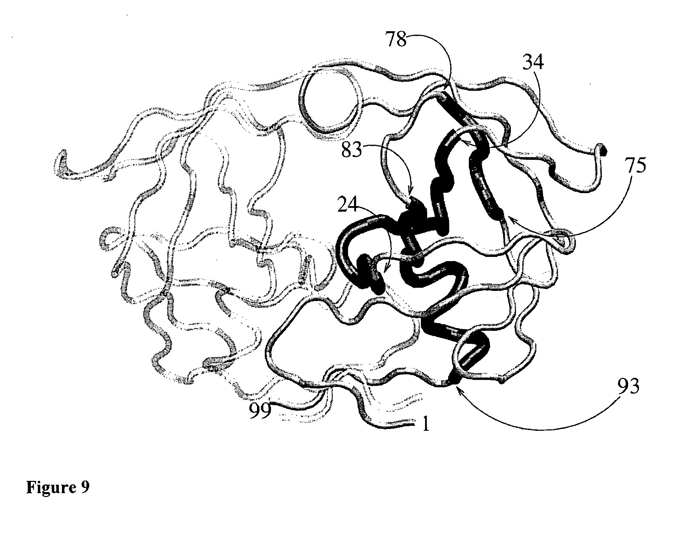

**Figure 9:** A cartoon of the native conformation of the HIV-1-PR homodimer. Each monomer contains 99 aa. Shown with different gray levels are the two monomers. In the monomer displayed to the right possible candidates of LES associated with this monomer are evidenced in dark gray [37].

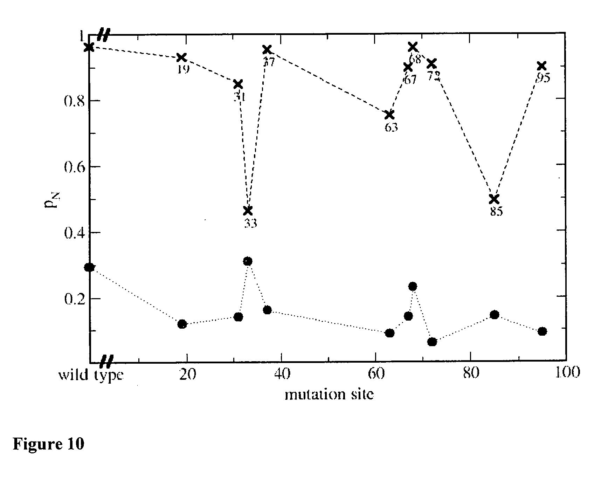

**Figure 10:** The effect of mutations on a number of sites of the monomer of the HIV-1-PR (x-axis) on the stability p_{N} of the native state of the protein (y-axis), calculated making use of a generalized C_{D} Gⓞ -model [37]. Solid crosses corresponds to the stability of the native conformation of the monomer alone (at the biological temperature T=2.5 kJ/mol), while the solid dots report the value of p_{N} of the monomer in presence of three p-LES of type p-S₈ (=83-93). The cross and closed dot drawn at mutation site=0 indicate the results associated with the wild-type sequence. The lines are to guide the eye.

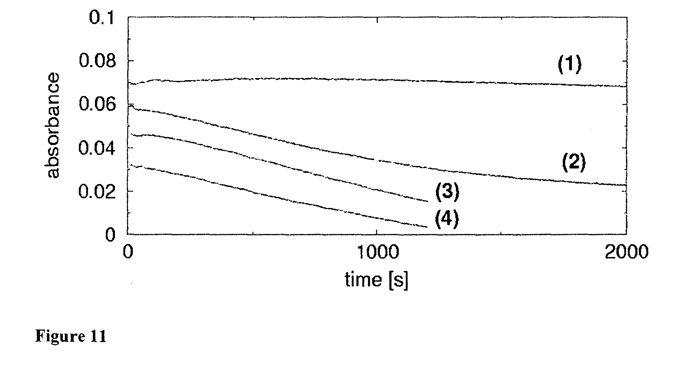

**Figure 11:** The absorbance of the activity benchmark of HIV-1-PR as a function of time in presence of the peptide 83-93 (1), of no inhibitor peptides (2), of peptides 61-70 (3) and peptides 9-19 (4) (from ref. [36]).

### Examples

### Example 1 (Src-SH3)

This domain displays five anti-parallel beta-sheets and an alpha-helix and is composed of 60 residues (see Fig. 1).

It is an interesting benchmark for the method of the invention as it has been widely characterized through thermodynamic as well as kinetic experiments [32].

Making use of a generalized Gⓞ model [33], simulations of the folding of the SH3 domain were carried out and the probability that the domain is in its native conformation calculated (see Fig. 2). We have repeated the calculations evolving in each case the SH3 domain in the presence of a peptide displaying identical sequence to that of one-of 28 segments of the protein of length N/10 (=6), namely 1-6, 3-8, 5-10, ..., 55-60, in the ratio peptide-domain 3:1. The results are shown in Figs. 3, 4 and 5.

From these results it can be clearly pointed out that folding is inhibited by peptides p-S₁=3-8, p-S₂=21-27, p-S₃=35-40 and p-S₄=45-50, the p-S₃ peptide being the most efficient.

Consequently, the segments *Sᵢ (i*=*1,2,3* and *4*) qualify as LES of the src SH3 domain.

The fact that peptides p-Sᵢ (*i*=*1,2,3* and *4*) are not only efficient inhibitors but also permanently effective is documented by the results displayed in Fig. 6.

In Fig. 6 it is reported the effect point mutations have on the stability of the protein by itself and in the presence of three p-S₃ peptides. Mutations which do not affect the stability nor the folding ability of the protein (e.g. mutation in sites #9 and #30) leave unchanged the inhibitory ability of the p-S₃ peptide. On the other hand, escape mutants (for example, mutations on site #(5,18,26,49 and 55)) are not able to fold. These results are consistent with the fact that sites #(9, 30), #(5,49,55) and #(18,26) qualify as cold, warm and hot sites, respectively.

By combining the results displayed in Fig. 5 and 7, one can slightly adjust the length and the initial and final amino acid number of the four inhibitors p-Sᵢ (i=1,2,3 and 4), in order to secure that they include all of the hot sites (sites #18,26,27 and 40) and most of the warm sites (4,5,6,28,39,41,48,49,50,55). A possible outcome of a first iteration carried out along these lines gives: p-S₁=p-S₁'=3-8 (containing the warm amino acid 4, 5 and 6), p-S'₂=18-28 (hot amino acid 18,26,27; warm amino acid 28), p-S'₃=36-42 (hot amino acid 40; warm amino acid 39, 41), p-S'₄=p-S₄=45-50 (warm amino acid 48, 49,50). It is noted that a single (warm) amino acid (#55) is not englobed/included by this set of inhibitor peptides. This in keeping with the fact that not all warm sites, participating in the (post-critical) FN, belong necessarily to LES (e.g., the warm amino acid #16 of the S₃₆-model protein is part of the post-critical FN but does not belong to any LES [16]). The improved efficiency of the iterated peptides is exemplified by the results displayed in Fig. 5 for p-S₂' and p-S₃' (open dots).

It is worth noting that all of the results reported until now emerge from model calculations. Because in the present case (Src-SH3 protein domain), detailed experimental information exist concerning the amino acids which play an important role in the folding process (that is one knows the ΔΔG values from protein engineering [32], see Fig. 8), it is possible to take advantage of this information to carry out the iteration process.

From the results displayed in Fig. 8 it is to be pointed out that the "hot" and "warm" sites correspond to amino acids #10,20,24,26 and to amino acids #5,7,18,23,38,41,48, 50, respectively.

Making use of these results, and of those displayed in Figs. 5 and 6, it is found that a possible first iteration gives p-S₁'=5-10 (containing the hot amino acid 10, and the warm amino acid #5, 7), p-S₂'=20-26 (hot amino acid #20,24,26; warm amino acid #23), p-S₃'=38-44 (warm amino acid #38,41,44) and p-S₄'=p-S₄=45-50 (warm amino acid #48,50).

The inhibitory properties of these peptides determined through the above approach should be tested through the assays quoted in step (d) of the method of the invention.

### Example 2 (HIV-PR, computational)

The HIV-1-PR is a homodimer formed by chains containing 99 aa each (Fig. 9). The stability properties of this enzyme has been studied through lengthy all-atom simulations over hundreds of nanoseconds (ns). Making use of the corresponding results a generalized 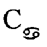 Gⓞ model was developed. It was then used to simulate the full dynamical evolution of the folding of the enzyme, and the results compared to those of all atom, standard Gⓞ model simulations available in the literature. Combining the insight obtained from these simulations and the information arising from mutations (Table 1) the "hot" and "warm" sites of the protein were determined and possible candidates of LES singled out. In particular, the region S₈=(83-93) (for details cf. [37] and refs. therein).

Simulations of the folding of the HIV-1-PR monomer were carried out in presence of three p-S₈ peptides. Defining the population *p_{N}* of the native state as the normalized probability that the chain displays a RMSD lower than 10Å and more than the 70% of native contacts formed, one finds *p_{N}*=0.28. This number has to be compared with *p_{N}*=0.87 for the protein alone under the same biological conditions, and to the numbers *p_{N}* =0.72 and 0.66 for the protein in the presence of control peptides having the same sequence as the fragment 61-71 and 4-14) [37].

Evidence for the fact that this inhibitor does not create resistance is shown in Fig. 10. Mutations which do not affect the stability nor the folding ability of the protein leave essentially unchanged the inhibitory properties of the p-S₈ peptide (e.g. mutation in site #19). Escape mutants (e.g., mutation in site #33) are essentially not able to fold.

### Example 3 (HIV-PR, experimental)

Peptides displaying a sequence identical to the segments 83-93 (S₈) and to the segments 9-19 and 61-70 of the wild-type HIV-1-PR monomer were obtained by solid-phase synthesis. Each solution was prepared adding 0.8 mM NaCl, 1nM EDTA and 1mM dithiothritol to a 20 mM phosphate buffer (pH 6), further adding 2.78 µg of HIV-1

Protease and 5.4 µM peptide (i.e., the concentration of each of the different peptides is 3 times that of the protease).

Spectroscopic assays were performed making use of a chromogenic substrate [34], measuring its change in absorbance with respect to time at 310 nm [36]. Some of the corresponding results are reported in Fig. 11. It is seen that peptide 83-93 decreases consistently the activity of the protease and can thus be used as inhibitor. This sequence can thus be interpreted as giving rise to a LES of the HIV-1-PR. As observed from Fig. 9, this LES is well structured, containing a number of internal native contacts (which stabilize an alpha-helix turn). It thus qualifies as a particularly specific peptide inhibitor. It is worth noting that mutations observed in this fragment [35] or in its complementary fragment (i.e. fragment 24-34), are found to be conservative mutations, whether induced by commercial drugs (aimed at inhibiting the active site of the protease) or not (cf. Table 1).

**Table 1: The observed mutations of the HIV-1-PR as reported in ref. [35]. For each residue of the wild-type sequence (wt) are listed the mutations observed in treated and/or untreated patients (mut) and the PAM250 score assocaited with the least conservative of these mutations (the PAM250 is a score derived from the analysis of amino acid replacements occuring among related proteins. It specifies a range of positive values for replacements which commonly occur among related proteins (conservative mutations) and zero or negative scores for unlikely replacements (non-conservative mutations)). In bold are reported the sites which undergo non-conservative mutations.**

| wt | mut | PAM | wt | mut | PAM | wt | mut | PAM | wt | mut | PAM |
|---|---|---|---|---|---|---|---|---|---|---|---|
| P1 | | | T26 | | | G51 | | | L76 | V | 2 |
| Q2 | | | C27 | | | G52 | | | V77 | I | 4 |
| I3 | V | 4 | A28 | | | P53 | L | 2 | G78 | | |
| T4 | | | D29 | | | I54 | VMLT | 0 | P79 | A | 1 |
| L5 | | | D30 | N | 2 | K55 | RII | 0 | T80 | | |
| W6 | | | T31 | | | V56 | | | P81 | T | 0 |
| Q7 | | | V32 | I | 4 | R57 | K | 3 | V82 | TAFIS | -1 |
| R8 | KQL | 1 | L33 | FVI | 2 | Q58 | E | 2 | N83 | | |
| P9 | | | E34 | DQANG | 0 | Y59 | | | I84 | V | 4 |
| L10 | FIRV | -3 | E35 | DG | 0 | D60 | E | 3 | 185 | V | 4 |
| V11 | IL | 2 | M36 | ILV | 2 | Q61 | ENII | 1 | C86 | | |
| T12 | SPAEIKN | 0 | S37 | DSTEKHC | -4 | 162 | V | 4 | R87 | K | |
| I13 | V | 4 | L38 | F | 2 | L63 | PSTACQM | -6 | N88 | DS | 1 |
| K14 | R | 3 | P39 | SQT | 0 | I64 | VLM | 2 | L89 | MVI | 2 |
| I15 | V | 4 | G10 | | | E65 | D | 3 | L90 | M | 4 |
| G16 | EA | 0 | R41 | KN | 0 | I66 | FV | 1 | T91 | | |
| G17 | E | 0 | W42 | | | C67 | FS | -4 | Q92 | KR | 1 |
| Q18 | II | 3 | K43 | RT | 0 | G68 | | | 193 | L | 2 |
| L19 | ITVQP | -3 | P44 | | | H69 | ICQYRN | 0 | G94 | | |
| K20 | MRTIV | -2 | K45 | IRN | -2 | K70 | RTE | 0 | bf A96 | SF | -3 |
| E21 | | | M46 | FILV | 0 | A71 | TVT | -1 | T96 | | |
| A22 | | | 147 | V | 4 | 172 | VTLMER | -2 | L97 | V | 2 |
| L23 | 1 | 2 | bf G48 | V | -1 | G73 | STCA | 1 | N98 | | |
| L24 | IVF | 2 | C49 | | | T74 | SAP | 0 | F99 | | |
| D25 | | | 150 | VL | 2 | V75 | I | 4 | | | |

### Example 4

Given a protein containing N amino acids, peptides of length L = (N/10) ± 2 may be prepared, each displaying the same sequence of a segment of the protein. Peptide #1 coincides with the segment starting with amino acid 1 and ending with amino acid L, peptide #2 coincides with segment (L/z+1)―[(1+z)L/z], ...,while the ith peptide coincides with the segment (iL/z+1)―[(i+z)L/z], with i=1,2,...iₘ, where iₘ=zN/L-z. Consequently, the maximum number of peptides to be produced is *iₘ*+1. The quantity *z* controls the overlap allowed between two consecutive peptides. The recommended values of *z*=*2,3* leading to 50% and 67% overlap.

Let us choose for the sake of (realistic) exemplification the values N=100, L=10, z=3. One then obtains iₘ=27 and 67% overlap. The number corresponding to the first and the last amino acid of each of the 28 possible peptides are collected in Table 2.

Let us correlate these results with the case of the monomer of the HIV-1-PR dimer and assume three different scenarios followed in the search of the peptides which inhibits folding: a) ordered search starting from amino acid (aa) 1⁻ and proceedings to aa 100 (i.e., starting from the N end and proceeding towards the C end), b) ordered search but in the inverse order (100 to 1, i.e., from C to N ends), c) random search starting from the N and from the C end as well as from the middle of the protein, then moving away from these region so as to cover the whole protein. In the first case one would need 26 tries to find the p-LES inhibitor (peptide #26 of Table 2), in the second case 3 tries, while in the third case 12 tries (see Table 2).

**Table 2: An example of division of the sequence of HIV-1-PR into peptides. The columns indicate, respectively, the identification number of the peptide, the index i (see text), the corresponding fragment in the HIV-1-PR sequence, and (in roman numbers) an example of a non-sequential inhibitory test, starting from the centre of the protein and the C and N terminal ends essentially simultaneously. In this case the search is concluded after twelve steps when the peptide #26 is tested.**

| *peptide* # | *i* | *interval* | *random* |
|---|---|---|---|
| 1 | - | 1-10 | III |
| 2 | 1 | 4-13 | VII |
| 3 | 2 | 8-17 | XI |
| 4 | 3 | 11-20 | |
| 5 | 4 | 14-23 | |
| 6 | 5 | 18-27 | |
| 7 | 6 | 21-30 | |
| 8 | 7 | 24-33 | |
| 9 | 8 | 28-37 | |
| 10 | 9 | 31-40 | |
| 11 | 10 | 34-43 | |
| 12 | 11 | 38-47 | X |
| 13 | 12 | 41-50 | V |
| 14 | 13 | 44-53 | I |
| 15 | 14 | 48-57 | II |
| 16 | 15 | 51-60 | VI |
| 17 | 16 | 54-63 | IX |
| 18 | 17 | 58-67 | |
| 19 | 18 | 61-70 | |
| 20 | 19 | 64-73 | |
| 21 | 20 | 68-77 | |
| 22 | 21 | 71-80 | |
| 23 | 22 | 74-83 | |
| 24 | 23 | 78-87 | |
| 25 | 24 | 81-90 | |
| 26 | 25 | 84-93 | XII |
| 27 | 26 | 87-97 | VIII |
| 28 | 27 | 91-100 | IV |

### References

1. A. L. Horwich and J. S. Weissman, Deadly Conformations-Protein Misfolding in Prion Disease, Cell 89 (1997) 499
2. D. R. Booth et al., Instability, unfolding and aggregation of human lysozyme variants underlying amyloid fibrillogenesis, Nature 385 (1997) 787
3. D. Sidransky and M. Hollstein, Clinical implications of the p53 gene. Annual Review of Medicine, 47 (1996) 285
4. A. Fersht, Structure and Mechanism in Protein Science, Freeman, New York (1999)
5. C. Branden and J. Tooze, Introduction to Protein Structure, Garland, New York (1999)
6. H. J. Dyson and P. E. Write, Peptide conformation and protein folding, Curr. Opin. Struct. Biol. 3 (1993) 60-65
7. L. C. Wu, R. Grandorri and J. Carey, Autonomous Subdomains in Protein Folding, Prot. Sci. 3 (1994) 396-371
8. Y. Bai, J. S. Mulue, L. Mayne and S. W. Englander, Primary structure effects on peptide group hydrogen exchange, Proteins 17 (1993) 75-86
9. R. Jaenicke, Protein folding: local structures, domains, subunits, and assemblies Biochem. 30 (1991) 3147-61
10. A. Wallqvist, G. W. Smithers and D. G. Covell, A cooperative folding unit in HIV-1 protease. Implications for protein stability and occurrence of drug-induced mutations, Prot. Engin. 11 (1998) 999-1005
11. R. A. Broglia and G. Tiana, Hierarchy of Events in the folding of model proteins, J. Chem. Phys. 114 (2001) 7267-7273
12. G. Tiana and R. A. Broglia, Statistical Analysis of Native Contact Formation in the Folding of Designed Model Proteins, J. Chem. Phys. 114 (2001) 2503-2507
13. V. I. Abkevich, A. M. Gutin and E. I. Shakhnovich, Specific nucleus as the transition state for protein folding, Biochem. 33 (1994) 10026-10032
14. R. A. Broglia, G. Tiana and R. Berera, Resistance proof, folding-inhibitor drugs, J. Chem. Phys. 118 (2003) 4754-4758
15. R. A. Broglia and G. Tiana, Reading the three--dimensional structure of a protein from its amino acid sequence, Proteins 45 (2001), 421-427
16. G. Tiana, R. A. Broglia, H. E. Roman, E. Vigezzi and E. I. Shakhnovich, Folding and Misfolding of Designed Protein-like Folding and Misfolding of Designed Protein-like Chains with Mutations, J. Chem. Phys. 108 (1998) 757-761
17. D. B. Wetlaufer, Nucleation, Rapid Folding, and Globular Intrachain Regions in Proteins, Proc. Natl. Acad. Sci. USA 70 (1973) 697-701
18. D. B. Wetlaufer, Folding of protein fragments, Adv. Prot. Chem. 34 (1981) 61-92
19. G. E. Schuly and R. H. Schirmer, Principles of Protein Structure, Springer, Heidelberg (1979)
20. G. E. Schulz, Domain motions in proteins, Curr. Opin. Struct. Biol. 1 (1991) 883-888
21. K. A. Dill, Theory for the folding and stability of globular proteins, Biochem. 24 (1985) 1501-1509
22. J. S. Richardson, The Anatomy and Taxonomy of Protein Structure, Adv. Prot. Chem. 34 (1981) 167-339
23. J. Janin and S. J. Wodak, Structural domains in proteins and their role in the dynamics of protein function, Prog. Biophys. Mol. Biol. 42 (1983) 21-78
24. D. S. Goodsell and A. J. Olson, Soluble proteins: size, shape and function, Trends Biochem. Sci. 18 (1993) 65-68
25. R. F. Doolittle, Reconstructing history with amino acid sequences, Prot. Sci. 1 (1992) 191-200
26. P. Bork, Mobile modules and motifs, Curr. Opin. Struct. Biol. 2 (1992) 413-421
27. A. L. Berman, E. Kolker and E. N. Trifonov, Underlying order in protein sequence organization, Proc. Natl. Acad. Sci. USA 91 (1994) 4044-4047
28. D. Xu and R. Nussinov, Favorable domain size in proteins, Fold. Design. 3 (1998) 11
29. E. Shakhnovich, Proteins with selected sequences fold into unique native conformation, Phys. Rev. Lett. 72 (1994) 3907
30. G. Tiana and R. A. Broglia, Folding and design of dimeric proteins, Proteins 49 (2002) 82-94
31. C. Cantor and C. Schimmel, Biophysical Chemistry, W. H. Freeman and Co. (1994)
32. V. P. Grantcharova, D. S. Riddle, J. V. Santiago and D. Baker, Important role of hydrogen bonds in the structurally polarized transition state for folding of the src SH3 domain, Nature Struct. Biol., 5 714 (1998)
33. N. Gⓞo, Theoretical studies of protein folding. Annu Rev Biophys Bioeng, 12 183-21 (1983)
34. T. A. Tomaszek et al. Chromophoric peptide substrates for the spectrophotometric assay of HIV-1 protease, Biochem. Biophys. Res. Comm 168, 274-280 (1990)
35. R. W. Shafer, P. Hu, A. K. Patick, C. Craig and V. Brendel, Identification of biased amino acid substitution patterns in human immunodeficiency virus type 1 isolates from patients treated with protease inhibitors, J. Virol. 73 (1999) 6197-6202
36. R. A. Broglia, G. Tiana, D. Provasi, F. Simona, L. Sutto, F. Vasile and M. Zanotti, to be published, Design of a folding inhibitor of the HIV-1 Protease, q-bio/0408013
37. R. A. Broglia, G. Tiana, L. Sutto, D. Provasi and F. Simona, Design of HIV-1-PR inhibitors which do not create resistance: blocking the folding of single monomers, q-bio/0504011
38. H. Maity, M. Maity, M. M. G. Krishna, L. Mayne and S. W. Englander, NMR characterization of residual structure in the dentaured state of proten L, Proc. Natl. Ac. Sci. USA, 102 (2005) 4741-4746
39. Q. Yi, M. L. Salley-Kim, E. J. Alm and D. Baker, NMR Characterization of residual structure in the denatured state of protein L, J. Mol. Biol. 299 (2000) 1341-1351.
40. R. A. Broglia, G. Tiana and D. Provasi, Simple models of protein folding, J. Phys. Cond. Mat. 16 (2004) R111-R114
41. A. M. Lesk and G. D. Rose, Folding units in globular proteins, Proc. Natl. Acad. Sci. USA (1981) 4304-4308

## Claims

1. A method for the identification of peptide inhibitors of the folding of a protein of N amino acids of length L, which method comprises:
a) designing M peptides of length L typically of the order of 10 and, in general, ranging from about 4 to about 20, each displaying a sequence identical to a segment of the protein under consideration so as to cover the entire protein length with some degree of overlap, wherein M is an integer varying typically from 5 to 50;
b) preparing the M designed peptides either singularly or in groups;
c) preparing M solutions each containing the protein under consideration and one of the peptides in a suitable molar ratio and incubating each of the solutions at 37°C;
d) assessing the inhibitory efficiency of the peptide in the above solutions or the degree of unfolding or both, by means of standard techniques, so as to identify the peptide endowed with inhibitory activity towards the protein.

2. The method of claim 1 wherein N is comprised from 60 to 150.

3. The method of claim 1 wherein the length L of the peptide is comprised between 4 and 20.

4. The method of claim 1 wherein the length of the peptide corresponds to L=N/10.

5. The method of claim 1 wherein step (b) is carried out by synthesizing the M peptides or by properly cutting the target protein, according to standard methods.

6. The method of claim 1 wherein the molar ratio peptide/protein may vary from 1:1 to 10:1.

7. The method of claim 5 wherein the molar ratio peptide/protein is 3 to 1.

8. The method of claim 1 wherein steps (b) and (c) can be substituted by computer programs simulations.

9. The method of claim 1 wherein step (b) and (c) is carried out through spectrophotometric assays, sedimentation equilibrium tests, circular dichroism or nuclear magnetic resonance techniques.

10. A peptide inhibitor being identified through a method as defined in any one of claims from 1 to 8.

11. A peptide inhibitor as defined in claim 9 for use as a medicament.

12. The method of claim 1 applied to each domain of multi-domain proteins.
